(19) Europäisches Patentamt

European Patent Office

Office européen des brevets

(11) **EP 2 990 395 B2**

(12) **NEW EUROPEAN PATENT SPECIFICATION**

After opposition procedure

(45) Date of publication and mention
of the opposition decision:
**01.06.2022 Bulletin 2022/22**

(45) Mention of the grant of the patent:
**10.01.2018 Bulletin 2018/02**

(21) Application number: **14788088.4**

(22) Date of filing: **23.04.2014**

(51) International Patent Classification (IPC):
*C07C 1/24* (2006.01)          *C07C 11/02* (2006.01)
*C07C 303/02* (2006.01)          *C07C 303/32* (2006.01)
*C07C 309/20* (2006.01)          *C07B 61/00* (2006.01)

(52) Cooperative Patent Classification (CPC):
(C-Sets available)
**C07C 1/24; C07C 303/32**          (Cont.)

(86) International application number:
**PCT/JP2014/061493**

(87) International publication number:
**WO 2014/175359 (30.10.2014 Gazette 2014/44)**

(54) **METHOD FOR PRODUCING OLEFIN**

VERFAHREN ZUR HERSTELLUNG VON OLEFIN

PROCÉDÉ DE PRODUCTION D'UNE OLÉFINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.04.2013 JP 2013090655**

(43) Date of publication of application:
**02.03.2016 Bulletin 2016/09**

(73) Proprietor: **Kao Corporation
Chuo-ku
Tokyo 103-8210 (JP)**

(72) Inventors:
• **TAKAMURA, Rena
Wakayama-shi
Wakayama 640-8580 (JP)**
• **TAKADA, Shingo
Wakayama-shi
Wakayama 640-8580 (JP)**

(74) Representative: **Hoffmann Eitle
Patent- und Rechtsanwälte PartmbB
Arabellastraße 30
81925 München (DE)**

(56) References cited:
EP-A1- 2 495 227          WO-A1-01/44145
WO-A1-2011/052732          WO-A1-2014/103898
DE-A1- 2 833 551          DE-A1- 3 915 493
GB-A- 917 047          GB-B- 2 146 323
JP-A- H02 306 956          JP-A- 2012 232 944
JP-C1- 145 452          US-A- 4 234 752

US-A- 4 260 845          US-A1- 2007 299 291
US-B2- 8 455 701

• NADIR B. GODREJ et al.: "Alpha-Olefins from
Oleochemial Raw materials: The Godrej-Lurgi
Pro- cess", Proceedings of the 3rd World
Conference on Detergents, 1994, pages 222-227,
• KIRK-OTHMER: "olefins, higher" In:
"Encyclopedia of Chemical technology", 1996
vol. 17, pages 839-858,
• "Temperature-Programmed Desorption (TPD)
For Characterizing The Acid Sites On Oxide
Surfaces Sponsored by Micromeritics Instrument
Corporation", azonano.com, 18 January 2006
(2006-01-18), Retrieved from the Internet:
URL:https://www.azonano.com/article.aspx?A
rticleID=1475
• Desorption diagram
• RICHARDSON, J.T. et al.: In: "Principles of
Catalyst Development", 1989, New York page 104,
• Tables A-F, Kao Corporation experimental report;
• Table E (comparative examples 9 and 10, example
10)
• Table A, comparative example 1, standard
deviations
• "Olefination of fatty alcohols", Sasol
experimental report;
• Safety Data Sheet "KALCOL 6098" (C16 alcohol);
• Safety Data Sheet "KALCOL 8098" (C18 alcohol);
• EC-SAFETY DATA SHEET "NAFOL 1618";
• "Fettsauren", volume 11, Ullmanns Encyklopädie
der technischen Chemie, 4th edition, pages
528-537

**(Cont. next page)**

EP 2 990 395 B2

- '0lefination of Fatty Alcohols - Repoducibility Experiments", Sasol experimental report;
- "Additional experimental data", experimental report.
- Olefination of fatty alcohols: Repetition of the catalysed Hexadecanol dehydration reaction; Sasol experimental report;
- Repetition of arguments filed with Section V.2 of submission of 13.03.2020"

(52) Cooperative Patent Classification (CPC): (Cont.)

C-Sets
C07C 1/24, C07C 11/02;
C07C 303/32, C07C 309/20

**Description**

Field of the Invention

The present invention relates to a method for producing an olefin.

Background of the Invention

Various methods have been known as a method for producing an olefin compound through dehydration reaction of an alcohol. For example, PTL 1 describes a method for producing an olefin compound through dehydration reaction of a primary alcohol in the presence of trifluoromethanesulfonic acid as a dehydration catalyst at a reaction temperature of from 200 to 250°C.
PTL 2 describes a method for producing an olefin compound through dehydration reaction of a tertiary alcohol in a gas phase in the presence of an aluminosilicate as a solid catalyst at a reaction temperature of from 200 to 400°C.
The present inventors have found that an olefin may be obtained in a short period of time with a high yield by performing liquid phase dehydration reaction of an alcohol by using such a catalyst as $\gamma$-alumina having particular weak acid sites, and had filed a patent application (see PTL 3).
PTL 4 describes a method for producing an olefin compound through dehydration reaction of a primary alcohol using an alumina catalyst in the presence of an amine.
PTL 5 describes a method for producing an olefin using two kinds of alcohols having different numbers of carbon atoms in the presence of a catalyst containing a metal or a chloride of zinc or iron.

Citation List

Patent Literatures

PTL 1: JP-T-2008-538206
PTL 2: JP-A-2008-56671
PTL 3: WO 2011/052732
PTL 4: JP-A-2003-95994
PTL 5: Japanese Patent No. 145,452

Summary of the Invention

The present invention relates to a method for producing an olefin, containing subjecting a raw material alcohol to dehydration reaction in the presence of an acid catalyst, in which the acid catalyst contains a metal oxide containing aluminum, the raw material alcohol contains two or more kinds of alcohols having different numbers of carbon atoms each being a primary aliphatic alcohol having 8 or more and 22 or less carbon atoms, and a temperature of the reaction is 200°C or more and 300°C or less, wherein the dehydration reaction is liquid phase reaction.

Technical Problem

However, the reaction using trifluoromethanesulfonic acid described in PTL 1 has a problem that the reaction is difficult to control.
The reaction using a silica alumina catalyst described in PTL 2 has a problem that an oligomerized product of an olefin is liable to be formed, and the yield of the target product may be reduced.
In the method described in PTL 3, the oligomerized product as a by-product may be suppressed, but the development of a further effective production method has been demanded.
In the method described in PTL 4, the selectivity of an $\alpha$-olefin in the olefin thus produced may be enhanced, but there is no description about the suppression of the by-product due to oligomerization, and an olefin is not obtained with a sufficient yield.
In the method of PTL 5, further improvement has been demanded since there is no investigation on the suppression of the oligomerized product, and the yield is not sufficient. Furthermore, there is such a description that the yield may be reduced in the case where a mixture of alcohols having two kinds of alkyl chain lengths is used as a raw material, as compared to the case where only one kind of an alcohol is used as a raw material, but there is no suggestion about a method for preventing the reduction of the yield due to the mixture.

The present invention relates to a method for producing an olefin with a high yield through dehydration reaction of an aliphatic alcohol while suppressing oligomerization of an olefin.

Solution to Problem

The inventors have found that oligomerization of an olefin can be suppressed, and thereby an olefin can be produced with a high yield, by performing dehydration reaction by combining a particular aliphatic alcohol.
The present invention relates to the following item [1].

[1] A method for producing an olefin, containing subjecting a raw material alcohol to dehydration reaction in the presence of an acid catalyst, the acid catalyst containing a metal oxide containing aluminum, the raw material alcohol containing two or more kinds of alcohols having different numbers of carbon atoms each being a primary aliphatic alcohol having 8 or more and 22 or less carbon atoms, and a reaction temperature being 200°C or more and 300°C or less, wherein the dehydration reaction is liquid phase reaction.

Advantageous Effect of Invention

The present invention can provide a method for producing an olefin with a high yield through dehydration reaction of an aliphatic alcohol while suppressing oligomerization of an olefin.

Detailed Description of the Invention

The method for producing an olefin of the present invention contains subjecting as a raw material alcohol two or more kinds of alcohols having different numbers of carbon atoms each being a primary aliphatic alcohol having 8 or more and 22 or less carbon atoms to reaction in the presence of an acid catalyst containing a metal oxide containing aluminum at a particular temperature, and thereby an olefin can be produced with a high yield.

Raw Material Alcohol

The raw material alcohol used in the present invention is two or more kinds of alcohols having different numbers of carbon atoms each being a primary aliphatic alcohol having 8 or more and 22 or less carbon atoms.
The reason why the use of the alcohols having different numbers of carbon atoms suppresses the oligomerization of the olefin to provide the olefin with a high yield is not clear but may be considered as follows.
It may be estimated that the unevenness in the number of carbon atoms of the raw material alcohol suppresses the mutual action among the molecules of the raw material alcohol and the resulting olefin, such as hydrophobic mutual action. It may be thus considered that the reaction among the molecules is suppressed, and thus the dimerization is suppressed.
The number of kinds of the alcohols used as raw material is preferably 5 kinds or less, more preferably 3 kinds of less, and further preferably 2 kinds, from the standpoint of obtaining the olefin with a high yield.
The number of carbon atoms of the alcohol used as the raw material is 8 or more, preferably 12 or more, more preferably 14 or more, and further preferably 16 or more, from the standpoint of obtaining the olefin with a high yield, and is 22 or less, preferably 20 or less, more preferably 18 or less, and further preferably 14 or less, from the standpoint of the suppression of the oligomerization of the olefin.
The number of carbon atoms of the alcohol used as the raw material is 8 or more and 22 or less, preferably 12 or more and 22 or less, and more preferably 12 or more and 18 or less, from the standpoint of obtaining the olefin with a high yield and the standpoint of suppressing the oligomerization of the olefin.
The number of carbon atoms of the alcohol used as the raw material is further preferably 14 or more and 18 or less, and still further preferably 16 or more and 18 or less, from the standpoint of obtaining the olefin with a high yield.
The number of carbon atoms of the alcohol used as the raw material is preferably 8 or more and 16 or less, more preferably 8 or more and 14 or less, and further preferably 12 or more and 14 or less, from the standpoint of the suppression of the oligomerization of the olefin.
Preferred examples of the raw material alcohol from the standpoint of obtaining the olefin with a high yield and the standpoint of suppressing the oligomerization of the olefin include two or more kinds selected from 1-octanol, 1-nonanol, 1- decanol 1-undecanol, 1-dodecanol, 1-tridecanol, 1-tetradecanol, 1-pentadecanol, 1-hexadecanol, 1-heptadecanol, 1-octadecanol, 1-nonadecanol, 1-eicosanol, 1-heneicosanol, and 1-docosanol, more preferably two or more kinds selected from 1-dodecanol, 1-tridecanol, 1-tetradecanol, 1-pentadecanol, 1-hexadecanol, 1-heptadecanol, 1-octadecanol, 1-nonadecanol, and 1-eicosanol, and further preferably two or more kinds selected from 1-dodecanol, 1-tetradecanol, 1-hexadecanol, and 1-octadecanol.

Preferred examples thereof from the standpoint of obtaining the olefin with a high yield include two or more kinds selected from 1-tetradecanol, 1-hexadecanol, and 1-octadecanol, and more preferably two kinds of 1-hexadecanol and 1-octadecanol.

Preferred examples thereof from the standpoint of the suppression of the oligomerization of the olefin include two or more kinds selected from 1-dodecanol, 1-tetradecanol, and 1-hexadecanol, and more preferably two kinds of 1-dodecanol and 1-tetradecanol.

In the case where two or more kinds of alcohols having different numbers of carbon atoms are used, the total content of the alcohol that has the first largest content and the alcohol that has the second largest content in the raw material alcohol is preferably 50% by mass or more, more preferably 70% by mass or more, further preferably 90% by mass or more, and still further preferably 100% by mass, from the standpoint of the usefulness of the resulting olefin.

The two kinds of alcohols that has the first and second largest contents are an alcohol (A) and an alcohol (B) having a larger number of carbon atoms than the alcohol (A), in which the mass ratio (A/B) of the alcohol (A) and the alcohol (B) is preferably 1/9 or more and 9/1 or less, more preferably 1/4 or more and 4/1 or less, further preferably 1/4 or more and 3/1 or less, still further preferably 1/2 or more and 2/1 or less, and still further preferably 2/3 or more and 3/2 or less, from the standpoint of the suppression of the oligomerization of the olefin.

The difference in the number of carbon atoms between the alcohol (A) and the alcohol (B) is preferably 1 or more and 4 or less, more preferably 1 or more and 3 or less, and further preferably 2, from the standpoint of the suppression of the oligomerization of the olefin.

In the present invention, the case where the number of carbon atoms of the alcohol (A) is 16 and the number of carbon atoms of the alcohol (B) is 18, particularly the case where the alcohol (A) is 1-hexadecanol and the alcohol (B) is 1-octadecanol, and the case where the alcohol (A) is 1-tetradecanol and the alcohol (B) is 1-octadecanol are preferred from the standpoint of obtaining the olefin with a high yield. Among these, the case where the alcohol (A) is 1-hexadecanol and the alcohol (B) is 1-octadecanol is more preferred.

The case where the alcohol (A) is 1-dodecanol and the alcohol (B) is 1-tetradecanol is preferred from the standpoint of the suppression of the oligomerization of the olefin.

Catalyst

In the present invention, an olefin is produced by subjecting primary aliphatic alcohols having 8 or more and 22 or less carbon atoms to dehydration reaction in the presence of an acid catalyst containing a metal oxide containing aluminum. In the present invention, an olefin may be produced by subjecting primary aliphatic alcohols having 12 or more and 22 or less carbon atoms to dehydration reaction in the presence of an acid catalyst containing a metal oxide containing aluminum.

Solid Acid Catalyst

The acid catalyst containing a metal oxide containing aluminum is preferably a solid acid catalyst from the standpoint of the enhancement of the reaction rate of the dehydration reaction while controlling the reaction safely and the standpoint of producing the olefin with a high efficiency by recovering and reusing the catalyst.

The ratio of the weak acid amount of the solid acid catalyst is preferably 60% or more, and more preferably 70% or more, from the standpoint of the suppression of the side reaction, and is preferably 100% or less, more preferably 99% or less, further preferably 90% or less, still further preferably 80% or less, and still further preferably 75% or less, from the standpoint of the enhancement of the reaction rate of the dehydration reaction.

The ratio of the weak acid amount referred in the description herein means an acid amount that is calculated from the ammonia desorption amount at a desorption temperature of 300°C or less based on the total acid amount, which are measured by an $NH_3$-TPD method.

The $NH_3$-TPD method is such a method that ammonia is adsorbed on a solid catalyst, the temperature of which is then continuously increased at a controlled constant temperature increasing rate, and the amount of ammonia desorbed and the desorption temperature are measured. The acid amount and the acid strength of the catalyst may be measured thereby since ammonia that is adsorbed to weak acid sites among the acid sites of the solid catalyst is desorbed at a lower temperature, whereas ammonia that is adsorbed to strong acid sites is desorbed at a higher temperature. The measurement by the $NH_3$-TPD method may be performed, for example, with a catalyst analyzer, Automatic Temperature Programmed Desorption Analyzer "TPD-1At", produced by Bel Japan, Inc.

The weak acid amount may be measured as a relative amount with respect to the high peak (i.e., the peak on the higher temperature side among the two peaks measured), which is assumed to be 0.99 mmol/g, with ZSM-5 type zeolite, JRC-Z5-25H, produced by ExxonMobil Catalyst Technologies LLC. The peaks are detected by quantitative determination of ammonia with the fragment of ammonia m/e = 16 in the mass spectrum.

The measurement method used for the $NH_3$-TPD method may be a measurement method that has been ordinarily performed. For example, the pretreatment, the $NH_3$ adsorption treatment and the vacuum treatment are performed under the following conditions, and then the TPD measurement is performed.

Pretreatment: The temperature is increased to 200°C over 20 minutes, and maintained for 1 hours, in helium.
$NH_3$ adsorption treatment: $NH_3$ is adsorbed at 50°C and 2.7 kPa for 10 minutes.
Vacuum treatment: treatment at 50°C for 4 hours with a degree of vacuum: 2.7 kPa
TPD measurement: Helium gas is fed at 50 mL/min, and the temperature is increased to 600°C at a temperature increasing rate of 5°C per minute.

In the present invention, the weak acid amount is calculated from the ammonia desorption amount in the temperature range of from the start of measurement to the desorption temperature of 300°C, the strong acid amount is calculated from the ammonia desorption amount in the temperature range of from the desorption temperature exceeding 300°C to the temperature where ammonia is completely desorbed, and the sum thereof is designated as the total acid amount. The ratio of the weak acid amount with respect to the total acid amount is calculated by the following expression.

$$(\text{ratio of weak acid amount (\%)}) = ((\text{weak acid amount (mmol/g)}) \, / \, (\text{total acid amount (mmol/g)})) \times 100$$

The weak acid amount in the solid acid catalyst is preferably 0.01 mmol/g or more, more preferably 0.05 mmol/g or more, and further preferably 0.1 mmol/g or more, from the standpoint of the suppression of the side reaction.
The acid catalyst containing a metal oxide contains aluminum, from the standpoint of the enhancement of the reaction rate of the dehydration reaction. Specifically, aluminum oxide is preferred, and γ-alumina is further preferred.
In the present invention, a solid acid catalyst containing aluminum oxide that supports an oxide other than aluminum oxide (which may be hereinafter referred to as an oxide-supported solid catalyst) may be used.
Examples of the element constituting the oxide other than aluminum oxide include an element having an electronegativity that is higher than aluminum. The value of the electronegativity referred in the present invention means the value of a Pauling electronegativity.
The electronegativity of the element is preferably 1.6 or more, more preferably 2.0 or more, and further preferably 2.4 or more, from the standpoint of the suppression of the side reaction, and is preferably 3.0 or less, and more preferably 2.6 or less, from the same standpoint.
The electronegativity of the element is preferably 1.6 or more and 3.0 or less, more preferably 2.0 or more and 3.0 or less, and further preferably 2.4 or more and 2.6 or less, from the standpoint of the suppression of the side reaction and the same standpoint.
Examples of the element having an electronegativity that is higher than aluminum (1.5) include one or more kind selected from sulfur (2.5), tungsten (1.7), phosphorus (2.1), silicon (1.8), molybdenum (1.8), iron (1.8), cobalt (1.8), nickel (1.8), copper (1.9), zinc (1.6), boron (2.0), gallium (1.6), indium (1.7), germanium (1.9), tin (1.8), antimony (1.9), bismuth (1.9) and selenium (2.4). Among these, one or more kind selected from sulfur, tungsten, phosphorus and silicon is preferred, and sulfur is more preferred, from the standpoint of the enhancement of the dehydration reaction rate. The values in the parentheses each are the value of the Pauling electronegativity.
Examples of a compound to be an oxide source of the element include an inorganic acid, such as sulfuric acid, thiosulfuric acid, phosphoric acid, tungstic acid and a silicic acid, and a salt thereof, an oxide, such as silica, an acidic metal alkoxide that is soluble in water through hydrolysis, such as tetramethyl orthosilicate and tetraethyl orthosilicate, and a heteropolyacid, such as silicotungstic acid and phosphotungstic acid, and a salt thereof.
As more specific compounds, examples of the inorganic acid and a salt thereof include sulfuric acid, sulfuric anhydride, ammonium sulfate, sodium sulfate, potassium sulfate, magnesium sulfate, calcium sulfate, sodium thiosulfate, ammonium thiosulfate, phosphoric acid, phosphorus pentoxide, ammonium phosphate, diammonium hydrogen phosphate, sodium phosphate, potassium phosphate, magnesium phosphate, calcium phosphate, tungstic acid, ammonium tungstate, sodium tungstate, potassium tungstate, calcium tungstate, ammonium paratungstate, ammonium metatungstate and tetramethylammonium silicate; examples of the oxide include colloidal silica, silica gel and water glass; examples of the acidic metal alkoxide include tetramethyl orthosilicate and tetraethyl orthosilicate; examples of the heteropolyacid and a salt thereof include silicotungstic acid, phosphotungstic acid, ammonium phosphotungstate, sodium phosphotungstate and potassium phosphotungstate.
Among these, an inorganic acid and a salt thereof, and an acidic metal alkoxide are preferred as the oxide source, sulfuric acid, ammonium sulfate, ammonium tungstate, diammonium hydrogen phosphate and tetraethyl orthosilicate are more preferred, and sulfuric acid is further preferred, from the standpoint of the enhancement of the dehydration reaction rate. The use of the catalyst may rapidly progress the dehydration reaction of the alcohol and may provide the target olefin with a high yield.

The amount of the oxide of the element supported on aluminum oxide is preferably 0.01 part by mass or more, more preferably 0.1 part by mass or more, further preferably 0.5 part by mass or more, still further preferably 0.8 part by mass or more, and still further preferably 1 part by mass or more, from the standpoint of the enhancement of the dehydration reaction rate, and is preferably 10 parts by or less, more preferably 8 parts by mass or less, further preferably 5 parts by mass or less, and still further preferably 3 parts by mass or less, from the standpoint of the suppression of the side reaction, such as the oligomerization of the olefin, all per 100 parts by mass of aluminum oxide.

When the amount of the oxide of the element supported on aluminum oxide is in the range, the reaction may be completed in a short period of time.

Preparation Method of Oxide-supported Solid Catalyst

The oxide-supported solid catalyst may be prepared by a method of evaporation to dryness, an adsorption method, an equilibrium adsorption method, a pore filling method, a spraying method, a precipitation method or the like.

Specific examples of the preparation method include a method, in which an aqueous suspension liquid or a water-containing solid of aluminum oxide, the oxide of the element, and an aqueous solvent are mixed to prepare an impregnated product, and the resulting impregnated product is dried and calcined. The aluminum oxide as the support can be obtained, for example, by a precipitation method, a sol-gel method or an alkoxide method, and the aluminum oxide used is preferably calcined at a temperature of 400°C or more, and more preferably 450°C or more, from the standpoint of the enhancement of the dehydration reaction rate through the sufficient crystallization of aluminum oxide, and is preferably calcined at a temperature of 900°C or less, more preferably 800°C or less, further preferably 700°C or less, still further preferably 600°C or less, and still further preferably 550°C or less, from the standpoint of the enhancement of the dehydration reaction rate through the provision of the sufficient surface area. The aluminum oxide used as the support in the present invention is preferably γ-alumina from the standpoint of the catalytic activity, i.e., the enhancement of the dehydration reaction rate.

The solvent or the aqueous solvent used in the aqueous suspension liquid is preferably water from the standpoint of the availability and the safety in the preparation process of the catalyst.

The solvent or the aqueous solvent used in the aqueous suspension liquid may contain an organic solvent that has compatibility with water, such as ethanol, isopropanol, methanol and acetone. In this case, the content of water in the solvent or the aqueous solvent used in the aqueous suspension liquid is preferably 50% by mass or more, more preferably 80% by mass or more, further preferably 90% by mass or more, and still further preferably 95% by mass or more, from the standpoint of the safety in the preparation process of the catalyst and the standpoint of the enhancement of the dehydration reaction rate.

The temperature at which the aluminum oxide is impregnated with the oxide of the element is preferably 0°C or more, and more preferably 20°C or more, and is preferably 100°C or less, more preferably 95°C or less, further preferably 90°C or less, still further preferably 50°C or less, and still further preferably 30°C or less, from the standpoint of the enhancement of the supporting rate and the standpoint of the enhancement of the dehydration reaction rate through the uniform support of the oxide of the element in the resulting catalyst.

The time of the impregnation is preferably 0.1 hour or more, more preferably 0.2 hour or more, and further preferably 0.5 hour or more, and is preferably 10 hours or less, more preferably 5 hours or less, and further preferably 2 hours or less, from the standpoint of the enhancement of the supporting rate and the standpoint of the enhancement of the dehydration reaction rate through the uniform support of the oxide of the element in the resulting catalyst.

The calcining temperature after the impregnation is preferably 300°C or more, more preferably 400°C or more, and further preferably 450°C or more, from the standpoint of the enhancement of the dehydration reaction rate through the formation of the oxide of the element, and is preferably 900°C or less, more preferably 850°C or less, further preferably 800°C or less, and still further preferably 550°C or less, from the standpoint of the enhancement of the dehydration reaction rate through the prevention of the reduction of the surface area of the catalyst and the maintenance of the degree of dispersion of the supported element.

The calcining time is preferably 1 hour or more, and more preferably 2 hours or more, from the standpoint of the enhancement of the dehydration reaction rate through the formation of the oxide of the element, and is preferably 10 hours or less, more preferably 5 hours or less, and further preferably 4 hours or less, from the standpoint of the enhancement of the dehydration reaction rate through the prevention of the reduction of the surface area of the catalyst and the maintenance of the degree of dispersion of the supported element.

The atmosphere for calcining is not particularly limited, and may be an inert gas atmosphere, an oxidizing atmosphere or a reducing atmosphere. The calcining may be performed in a closed condition or in a gas-flowing condition. The atmosphere is preferably a gas-flow condition of air or oxygen from the standpoint of the enhancement of the dehydration reaction rate through the oxidation of the supported element.

The catalyst thus obtained may be in an aggregated state and thus may be appropriately pulverized into the form of powder, particles or formed into noodles, pellets or the like, before use.

Properties of Catalyst

In the case where the catalyst is in the form of powder, the average particle diameter of the powder is preferably 1 $\mu$m or more, more preferably 5 $\mu$m or more, and further preferably 10 $\mu$m or more, from the standpoint of the enhancement of the production efficiency of the olefin through the facility of the recovery and reuse of the catalyst, and is preferably 300 $\mu$m or less, more preferably 250 $\mu$m or less, and further preferably 200 $\mu$m or less, from the standpoint of the sufficient exhibition of the catalytic activity of the catalyst.

The BET specific surface area of the catalyst is preferably 100 m$^2$/g or more, more preferably 120 m$^2$/g or more, and further preferably 140 m$^2$/g or more, from the standpoint of the sufficient exhibition of the catalytic activity of the catalyst, and is preferably 500 m$^2$/g or less, more preferably 400 m$^2$/g or less, and further preferably 300 m$^2$/g or less, from the standpoint of the retention of the strength of the catalyst activity during the reaction.

The average pore diameter of the catalyst is preferably 5 nm or more, more preferably 7 nm or more, and further preferably 9 nm or more, from the standpoint of the sufficient exhibition of the catalytic activity of the catalyst, and is preferably 50 nm or less, more preferably 40 nm or less, and further preferably 25 nm or less, from the standpoint of the maintenance of the strength of the catalyst during the reaction.

The pore volume of the catalyst is preferably 0.20 mL/g or more, more preferably 0.25 mL/g or more, and further preferably 0.30 mL/g or more, from the standpoint of the sufficient exhibition of the catalytic activity of the catalyst, and is preferably 2.0 mL/g or less, more preferably 1.5 mL/g or less, and further preferably 1.2 mL/g or less, from the standpoint of the maintenance of the strength of the catalyst during the reaction.

The average particle diameter of the catalyst is a median diameter that is measured in such a manner that 0.05 g of the catalyst is dispersed under stirring in ethanol (Cica-First Grade Reagent, produced by Kanto Chemical Co., Inc.) as a measuring solvent and measured for the median diameter with a laser diffraction scattering particle size distribution analyzer "LA-920" (produced by Horiba, Ltd.) (stirring rate: level 4) assuming that the refractive index is 1.10.

The BET specific surface area, the average pore diameter, and the pore volume can be measured by the following manners. By using a surface area and pore distribution analyzer "ASAP2020" (produced by Micromeritics Instrument Corporation), a specimen is subjected to a heating pretreatment at 250°C for 5 hours, and then measured for the BET specific surface area by a multi-point method using liquid nitrogen to provide the value within a range where the parameter C is positive.

The pore volume can be calculated by a BJH method (Barrett-Joyner-Halenda method), and the peak top of the pore distribution is designated as the average pore diameter. The BJH method referred herein is a method using cylindrical pores that each is not connected to another pore, in which the pore distribution is obtained by the capillary condensation and the multilayer adsorption of nitrogen gas. The details thereof are described in "Shimadzu Review", vol. 48, No. 1, pp. 35 to 44 (1991).

Amount of Catalyst Used

The amount of the catalyst used in suspension bed reaction is preferably 0.5% by mass or more, more preferably 1% by mass or more, further preferably 3% by mass or more, and still further preferably 4% by mass or more, from the standpoint of the enhancement of the dehydration reaction rate, and is preferably 20% by mass or less, more preferably 15% by mass or less, further preferably 10% by mass or less, still further preferably 7% by mass or less, and still further preferably 6% by mass or less, from the standpoint of the economy, all based on the raw material alcohol. When the amount of the catalyst used is in the range, the reaction temperature can be suppressed low while preventing the catalyst from being used excessively, thereby providing economic advantages.

Organic Solvent

In the production method of the present invention, an organic solvent may be used depending on necessity. The organic solvent that may be used in the present invention is not particularly limited as far as the solvent is in a liquid state at the reaction temperature, is compatible with the substrates and the product, and does not impair the reaction, and may be a mixture. The organic solvent is preferably such a one that may be isolated from the product after the reaction, by utilizing the difference in boiling point.

Examples of the organic solvent that may be used in the present invention include a hydrocarbon organic solvent, such as a saturated aliphatic hydrocarbon, an unsaturated aliphatic hydrocarbon and an aromatic hydrocarbon.

Dehydration Reaction (Olefination Reaction)

The reaction in the method of the present invention is dehydration reaction of an alcohol, and there is a possibility that the reaction rate thereof is decreased when by-produced water is accumulated in the reaction system. Accordingly, from the standpoint of the enhancement of the reaction rate, the reaction is preferably performed while removing the produced water outside the reaction system while introducing an inert gas, such as nitrogen or argon, into the reaction system at ordinary pressure under stirring, or under an absolute pressure of preferably 0.07 MPa or less, more preferably 0.05 MPa or less, and further preferably 0.01 MPa or less.

The reaction temperature is the boiling point of the raw material alcohol or less, is 200°C or more, preferably 230°C or more, more preferably 240°C or more, further preferably 250°C or more, still further preferably 260°C or more, still further preferably 270°C or more, and still further preferably 275°C or more, from the standpoint of the reaction rate and the standpoint of the suppression of the side reaction, such as oligomerization, and is 300°C or less, preferably 290°C or less, more preferably 285°C or less, and further preferably 280°C or less, from the standpoint of the energy consumption and the standpoint of the heat resistance of the production equipment.

The reaction temperature is 200°C or more and 300°C or less, preferably 230°C or more and 290°C or less, more preferably 240°C or more and 285°C or less, and further preferably 240°C or more and 280°C or less, from the standpoint of the reaction rate, the standpoint of the suppression of the side reaction, such as oligomerization, the standpoint of the energy consumption, and the standpoint of the heat resistance of the production equipment.

The reaction temperature is preferably 230°C or more and 300°C or less, more preferably 240°C or more and 300°C or less, further preferably 250°C or more and 300°C or less, still further preferably 260°C or more and 300°C or less, still further preferably 270°C or more and 290°C or less, and still further preferably 275°C or more and 285°C or less, from the standpoint of the reaction rate and the standpoint of the suppression of the side reaction, such as oligomerization.

In the present invention, the olefination reaction is performed as liquid phase reaction. The liquid phase reaction herein means reaction performed at a temperature that is boiling point of the raw material alcohol or less and the melting point thereof or more, i.e., a temperature, at which a liquid phase is present. In the case of the liquid phase reaction performed, the production cost may be suppressed since the raw materials may not be necessarily evaporated entirely. Furthermore the target product can be obtained with a high yield since the oligomerization of the olefin can be suppressed.

The reaction time is preferably such a period that provides an alcohol conversion of 95% or more, more preferably 97% or more, and further preferably 98% or more, from the standpoint of the yield of the target olefin. While such a reaction time may vary depending on the reaction temperature, the kind of the organic solvent, the kind and the amount of the catalyst used, and the like, the reaction time in suspension bed batch reaction is preferably 1 hour or more, more preferably 3 hours or more, further preferably 6 hours or more, and still further preferably 8 hours or more, and is preferably 50 hours or less, more preferably 40 hours or less, further preferably 35 hours or less, still further preferably 15 hours or less, and still further preferably 10 hours or less, from the standpoint of the yield of the olefin.

According to the production method of the present invention, the yield amount of the olefin with respect to the raw material alcohol, i.e., the yield of the olefin, is generally 90% or more. The formation rate of the dimer contained in the olefin is generally 5% or less.

In the method of the present invention, a crossover dimer formed through crossover dimerization of two or more kinds of alcohols having different numbers of carbon atoms may be contained as the dimer in some cases, and the crossover dimer can be detected by GC-MS.

In the present invention, only the olefin may be purified by distillation from the resulting reaction product obtained by the aforementioned process. The olefin having high purity obtained through distillation purification is useful as a raw material or an intermediate of a surfactant, such as an olefin sulfonate, an organic solvent, a softener, a sizing agent, and the like.

Method for producing Olefin Sulfonate

The method for producing an olefin sulfonate of an embodiment contain the following steps 1 to 3:

　　step 1: a step of sulfonating an olefin that is produced by the method for producing an olefin of the present invention,
　　step 2: a step of neutralizing a sulfonated product obtained in the step 1, and
　　step 3: a step of hydrolyzing a neutralized product obtained in the step 2.

Step 1: Sulfonating Step

The step 1 is a step of sulfonating an olefin that is produced by the method for producing an olefin of the present invention, and a sulfonated product is obtained in the step 1.

The sulfonation reaction in the step 1 may be performed by reacting 1 mol of the olefin with sulfur trioxide gas or sulfuric anhydride in an amount of preferably 1 mol or more and 1.2 mol or less.

The reaction temperature in the sulfonation reaction is preferably 20°C or more and 40°C or less from the standpoint of the yield.

The sulfonation reaction may be performed by using sulfuric anhydride in the form of liquid instead of sulfur trioxide gas under a condition of a reaction temperature of 0°C or more and 10°C or less.

Step 2: Neutralizing Step

The step 2 is a step of neutralizing the sulfonated product obtained in the step 1, and a neutralized product of the sulfonated product is obtained in the step 2.

The neutralization in the step 2 may be performed by reacting with an alkali aqueous solution containing an alkali in an amount of 1 time by mol or more and 1.5 times by mol or less with respect to the theoretical value of the sulfonic acid group.

Examples of the alkali aqueous solution capable of being used for the neutralization include a sodium hydroxide aqueous solution, a potassium hydroxide aqueous solution, an ammonia aqueous solution and a 2-aminoethanol aqueous solution, and a sodium hydroxide aqueous solution is preferred from the standpoint of the availability.

Step 3: Hydrolyzing Step

The step 3 is a step of hydrolyzing the neutralized product obtained in the step 2, and an olefin sulfonate is obtained in the step 3.

The hydrolysis treatment in the step 3 may be performed by reacting in the presence of water at a temperature of 90°C or more and 200°C or less for a period of 30 minutes or more and 4 hours or less.

The sulfonation reaction and the neutralization reaction may be performed continuously. After completing the neutralization reaction, the product may be purified by extraction, rinsing and the like.

In addition to the aforementioned embodiments, the present invention relates to the following methods for producing an olefin.

<1> A method for producing an olefin, containing subjecting a raw material alcohol to dehydration reaction in the presence of an acid catalyst, the acid catalyst containing a metal oxide, the raw material alcohol containing two or more kinds of alcohols having different numbers of carbon atoms each being a primary aliphatic alcohol having 8 or more and 22 or less carbon atoms, and a reaction temperature being 200°C or more and 300°C or less, wherein the dehydration reaction is liquid phase reaction.

<2> A method for producing an olefin, containing subjecting a raw material alcohol to dehydration reaction in the presence of an acid catalyst, the acid catalyst containing a metal oxide, the raw material alcohol containing two or more kinds of alcohols having different numbers of carbon atoms each being a primary aliphatic alcohol having 12 or more and 22 or less carbon atoms, and a reaction temperature being 200°C or more and 300°C or less.

<3> The method for producing an olefin according to the item <1> or <2>, wherein the total content of the alcohol that has the first largest content and the alcohol that has the second largest content in the raw material alcohol is preferably 50% by mass or more, more preferably 70% by mass or more, further preferably 90% by mass or more, and still further preferably 100% by mass.

<4> The method for producing an olefin according to any one of the items <1> to <3>, wherein the alcohol that has the first largest content and the alcohol that has the second largest content in the raw material alcohol are an alcohol (A) and an alcohol (B) having a larger number of carbon atoms than the alcohol (A), and the difference in the number of carbon atoms between the alcohol (A) and the alcohol (B) is preferably 1 or more and 4 or less, more preferably 1 or more and 3 or less, and further preferably 2.

<5> The method for producing an olefin according to any one of the items <1> to <4>, wherein the alcohol that has the first largest content and the alcohol that has the second largest content in the raw material alcohol are an alcohol (A) and an alcohol (B) having a larger number of carbon atoms than the alcohol (A), and the mass ratio (A/B) of the alcohol (A) and the alcohol (B) is preferably 1/9 or more and 9/1 or less, more preferably 1/4 or more and 4/1 or less, further preferably 1/4 or more and 3/1 or less, still further preferably 1/2 or more and 2/1 or less, and still further preferably 2/3 or more and 3/2 or less.

<6> The method for producing an olefin according to any one of the items <1> to <5>, wherein the number of carbon atoms of the alcohol used as the raw material is 8 or more, preferably 12 or more, more preferably 14 or more, and further preferably 16 or more.

<7> The method for producing an olefin according to any one of the items <1> to <6>, wherein the number of carbon atoms of the alcohol used as the raw material is 22 or less, preferably 20 or less, more preferably 18 or less, and further preferably 14 or less.

<8> The method for producing an olefin according to any one of the items <1> to <7>, wherein the number of carbon atoms of the alcohol used as the raw material is 8 or more and 22 or less, preferably 12 or more and 22 or less, more preferably 12 or more and 18 or less, further preferably 14 or more and 18 or less, and still further preferably 16 or more and 18 or less.

<9> The method for producing an olefin according to any one of the items <1> to <8>, wherein the number of carbon atoms of the alcohol used as the raw material is preferably 8 or more and 16 or less, more preferably 8 or more and 14 or less, and further preferably 12 or more and 14 or less.

<10> The method for producing an olefin according to any one of the items <1> to <9>, wherein the raw material alcohol is two or more kinds selected from 1-octanol, 1-nonanol, 1-decanonl, 1-undecanol, 1-dodecanol, 1-tridecanol, 1-tetradecanol, 1-pentadecanol, 1-hexadecanol, 1-heptadecanol, 1-octadecanol, 1-nonadecanol, 1-eicosanol, 1-heneicosanol, and 1-docosanol, more preferably two or more kinds selected from 1-dodecanol, 1-tridecanol, 1-tetradecanol, 1-pentadecanol, 1-hexadecanol, 1-heptadecanol, 1-octadecanol, 1-nonadecanol, and 1-eicosanol, further preferably two or more kinds selected from 1-dodecanol, 1-tetradecanol, 1-hexadecanol, and 1-octadecanol, still further preferably two or more kinds selected from 1-tetradecanol, 1-hexadecanol, and 1-octadecanol, and still further preferably two kinds of 1-hexadecanol and 1-octadecanol.

<11> The method for producing an olefin according to any one of the items <1> to <10>, wherein the raw material alcohol is two or more kinds selected from 1-dodecanol, 1-tetradecanol, and 1-hexadecanol, and more preferably two kinds of 1-dodecanol and 1-tetradecanol.

<12> The method for producing an olefin according to any one of the items <1> to <11>, wherein the number of kinds of the alcohols used as raw material is preferably 5 kinds or less, more preferably 3 kinds of less, and further preferably 2 kinds.

<13> The method for producing an olefin according to any one of the items <2> to <10> and <12>, wherein the number of carbon atoms of the alcohol (A) is 16 and the number of carbon atoms of the alcohol (B) is 18, and preferably the alcohol (A) is 1-hexadecanol and the alcohol (B) is 1-octadecanol.

<14> The method for producing an olefin according to any one of the items <2> to <12>, wherein the alcohol (A) is 1-dodecanol and the alcohol (B) is 1-tetradecanol.

<15> The method for producing an olefin according to any one of the items <1> to <14>, wherein the acid catalyst is a solid acid catalyst.

<16> The method for producing an olefin according to any one of the items <1> to <15>, wherein the acid catalyst is a metal oxide containing aluminum, preferably aluminum oxide, and more preferably γ-alumina.

<17> The method for producing an olefin according to any one of the items <1> to <16>, wherein the acid catalyst is a solid acid catalyst containing aluminum oxide that supports an oxide other than aluminum oxide.

<18> The method for producing an olefin according to the item <17>, wherein the electronegativity of the element constituting the oxide other than aluminum oxide is preferably 1.6 or more, more preferably 2.0 or more, and further preferably 2.4 or more, is preferably 3.0 or less, and more preferably 2.6 or less, and is preferably 1.6 or more and 3.0 or less, more preferably 2.0 or more and 3.0 or less, and further preferably 2.4 or more and 2.6 or less.

<19> The method for producing an olefin according to the item <17>, wherein the element constituting the oxide other than aluminum oxide is preferably one or more kind selected from sulfur, tungsten, phosphorus, silicon, molybdenum, iron, cobalt, nickel, copper, zinc, boron, gallium, indium, germanium, tin, antimony, bismuth and selenium, and the element constituting the oxide other than aluminum oxide is more preferably one or more kind selected from sulfur, tungsten, phosphorus and silicon, and further preferably sulfur.

<20> The method for producing an olefin according to any one of the items <17> to <19>, wherein the amount of the oxide other than aluminum oxide supported on aluminum oxide is preferably 0.01 part by mass or more, more preferably 0.1 part by mass or more, further preferably 0.5 part by mass or more, still further preferably 0.8 part by mass or more, and still further preferably 1 part by mass or more, and is preferably 10 parts by or less, more preferably 8 parts by mass or less, further preferably 5 parts by mass or less, and still further preferably 3 parts by mass or less, all per 100 parts by mass of aluminum oxide.

<21> The method for producing an olefin according to any one of the items <1> to <20>, wherein the amount of the catalyst used in suspension bed reaction is preferably 0.5% by mass or more, more preferably 1% by mass or more, further preferably 3% by mass or more, and still further preferably 4% by mass or more, and is preferably 20% by mass or less, more preferably 15% by mass or less, further preferably 10% by mass or less, still further preferably 7% by mass or less, and still further preferably 6% by mass or less, all based on the raw material alcohol.

<22> The method for producing an olefin according to any one of the items <1> to <21>, wherein the reaction temperature is the boiling point of the raw material alcohol or less, may be 200°C or more, preferably 230°C or more, more preferably 240°C or more, further preferably 250°C or more, still further preferably 260°C or more, still further preferably 270°C or more, and still further preferably 275°C or more, and may be 300°C or less, preferably 290°C or less, more preferably 285°C or less, and further preferably 280°C or less.

<23> The method for producing an olefin according to any one of the items <1> to <21>, wherein the reaction temperature may be 200°C or more and 300°C or less, preferably 230°C or more and 290°C or less, more preferably 240°C or more and 285°C or less, and further preferably 240°C or more and 280°C or less.

<24> The method for producing an olefin according to any one of the items <1> to <21>, wherein the reaction temperature is preferably 230°C or more and 300°C or less, more preferably 240°C or more and 300°C or less, further preferably 250°C or more and 300°C or less, still further preferably 260°C or more and 300°C or less, still further preferably 270°C or more and 290°C or less, and still further preferably 275°C or more and 285°C or less.

<25> The method for producing an olefin according to any one of the items <1> to <24>, wherein the dehydration reaction is performed while removing water produced through the dehydration reaction outside the reaction system.

<26> The method for producing an olefin according to any one of the items <1> to <25>, wherein the dehydration reaction is performed while introducing an inert gas.

<27> The method for producing an olefin according to any one of the items <1> to <26>, wherein the reaction time is preferably 1 hour or more, more preferably 3 hours or more, further preferably 6 hours or more, and still further preferably 8 hours or more, and is preferably 50 hours or less, more preferably 40 hours or less, further preferably 35 hours or less, still further preferably 15 hours or less, and still further preferably 10 hours or less.

<28> The method for producing an olefin according to any one of the items <1> to <27>, wherein the yield of the olefin is 90% or more, and the formation rate of the dimer contained in the olefin is 5% or less.

Example

Preparation of Catalyst

1.02 g (0.011 mol) of sulfuric acid (guaranteed reagent 95%, produced by Sigma-Aldrich Corporation) was added to 35.9 g of ion exchanged water to prepare an impregnation liquid. Subsequently, the impregnation liquid was added dropwise to 50.0 g of γ-alumina (Neobead GB-13, produced by Mizusawa Industrial Chemicals, Ltd.) and uniformly absorbed thereto. The mixture was allowed to stand at room temperature for 1 hour, then dried at 120°C, and calcined at 500°C for 3 hours in the air. The resulting catalyst had a weak acid amount of 73% measured by the $NH_3$-TPD method and an amount of sulfur supported of 2 parts by mass per 100 parts by mass of aluminum oxide as calculated from the charged amounts of the raw materials.

Example 1

Olefination Reaction

In a 300 mL four-neck flask equipped with a stirrer, 40.0 g (0.16 mol) of 1-hexadecanol (Kalcol 6098, produced by Kao Corporation), 160.0 g (0.59 mol) of 1-octadecanol (Kalcol 8098, produced by Kao Corporation) and 10.0 g (5% by mass based on the alcohols) of an alumina catalyst (Neobead GB-13, produced by Mizusawa Industrial Chemicals, Ltd., ratio of weak acid amount by $NH_3$-TPD method: 71%) were charged, and reacted for 9 hours under stirring at 280°C with nitrogen flowing in the system (nitrogen flow amount: 100 mL/min).

The solution after completing the reaction was diluted with hexane and determined for the products by analyzing with a gas chromatography analyzer (HP6890, produced Hewlett-Packard Company) equipped with a column (Ultra Alloy-1 Capillary Column 30.0 mm x 250 μm, produced by Frontier Laboratories Ltd.) and a detector (hydrogen flame ionization detector (FID)) under conditions of an injection temperature of 300°C, a detector temperature of 350°C and a He flow rate of 4.6 mL/min. The results are shown in Table 1.

The yield of the olefin was calculated according to the following expression.

$$\text{yield of olefin (\%)} = ((\text{amount of olefin (mol)}) / (\text{charged amount of raw material alcohol (mol)})) \times 100$$

Examples 2 to 10 and Comparative Examples 1 to 10

Olefination Reaction

The reaction was performed in the same manner as in Example 1 except that the amounts of the raw materials, the catalyst and the reaction conditions were changed as shown in Table 1, and the products after completing the reaction were measured. The reactions conditions and the results are shown in Tables 1 and 2.

Comparative Example 11

In a 300 mL four-neck flask equipped with a stirrer, 40.0 g (0.16 mol) of 1-hexadecanol (Kalcol 6098, produced by Kao Corporation), 160.0 g (0.59 mol) of 1-octadecanol (Kalcol 8098, produced by Kao Corporation) and 2.0 g of a 25% by mass aqueous solution of trifluoromethanesulfonic acid (produced by Wako Pure Chemical Industries, Ltd.) as a catalyst were charged, and tried to be reacted under stirring at 240°C in the same manner as in Example 1, but the reaction was stopped since intense heat generation and bumping occurred on increasing the temperature.

The details of the catalysts shown in Tables 1 and 2 are as follows. $\gamma$-Al$_2$O$_3$: Neobead GB-13 (produced by Mizusawa Industrial Chemicals, Ltd.) $\gamma$-Al$_2$O$_3$ powder: Neobead GB-20 (produced by Mizusawa Industrial Chemicals, Ltd.)

SO$_4$/$\gamma$-Al$_2$O$_3$: catalyst obtained in the preparation of the catalyst
silica-alumina: N632L (produced by JGC Corporation)
trifluoromethanesulfonic acid: produced by Wako Pure Chemical Industries, Ltd.

Table 1

| | | Example | | | | | | | | | |
|---|---|---|---|---|---|---|---|---|---|---|---|
| | | 1 | 2 | 3 | 4 | 5 | 6 | 7 | 8 | 9 | 10 |
| Reaction conditions | Catalyst | $\gamma$-Al$_2$O$_3$ | $\gamma$-Al$_2$O$_3$ | $\gamma$-Al$_2$O$_3$ | $\gamma$-Al$_2$O$_3$ | $\gamma$-Al$_2$O$_3$ | SO$_4$/ $\gamma$-Al$_2$O$_3$ | $\gamma$-Al$_2$O$_3$ | $\gamma$-Al$_2$O$_3$ | silica-alumina | $\gamma$-Al$_2$O$_3$ powder |
| | Ratio of weak acid amount (%) | 71 | 71 | 71 | 71 | 71 | 73 | 71 | 71 | 65 | 71 |
| | Raw material alcohol (A) | hexa-decanol | hexa-decanol | hexa-decanol | hexa-decanol | tetra-decanol | hexa-decanol | hexa-decanol | hexa-decanol | hexa-decanol | dodecanol |
| | Raw material alcohol (B) | octa-decanol | octa-decanol | octa-decanol | octa-decanol | octa-decanol | octa-decanol | octa-decanol | octa-decanol | octa-decanol | tetra-decanol |
| | Mass ratio (A/B) | 20/80 | 50/50 | 50/50 | 80/20 | 50/50 | 50/50 | 90/10 | 50/50 | 80/20 | 80/20 |
| | Reaction temperature (°C) | 280 | 280 | 260 | 280 | 250 | 280 | 280 | 250 | 260 | 240 |
| | Reaction time (hour) | 9 | 9 | 30 | 9 | 30 | 5 | 9 | 30 | 1 | 19 |
| Results | Alcohol conversion (%) | 100 | 100 | 100 | 100 | 99 | 100 | 100 | 99 | 58 | 96 |
| | Yield of olefin (%) | 97.3 | 98.6 | 95.5 | 96.8 | 50.0 | 97.4 | 96.3 | 50.8 | 50.3 | 68.5 |
| | Yield of dimer (%) | 1.6 | 1.2 | 3.0 | 2.8 | 4.9 | 2.6 | 3.7 | 4.2 | 7.5 | 0.7 |

Table 2

| | | Comparative Example | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Reaction conditions | Catalyst | $\gamma$-Al$_2$O$_3$ | $\gamma$-Al$_2$O$_3$ | $\gamma$-Al$_2$O$_3$ | $\gamma$-Al$_2$O$_3$ | SO$_4$/ $\gamma$-Al$_2$O$_3$ | SO$_4$/ $\gamma$-Al$_2$O$_3$ | silica-alumina | silica-alumina | $\gamma$-Al$_2$O$_3$ powder | $\gamma$-Al$_2$O$_3$ powder | trifluoro-methane-sulfonic acid |
| | Ratio of weak acid amount (%) | 71 | 71 | 71 | 71 | 73 | 73 | 65 | 65 | 71 | 71 | - |
| | Raw material alcohol (A) | octa-decanol | hexa-decanol | hexa-decanol | tetra-decanol | octa-decanol | hexa-decanol | octa-decanol | hexa-decanol | dodecanol | tetra-decanol | hexa-decanol |
| | Raw material alcohol (B) | - | - | - | - | - | - | - | - | - | - | octa-decanol |
| | Mass ratio (A/B) | 100/0 | 100/0 | 100/0 | 100/0 | 100/0 | 100/0 | 100/0 | 100/0 | 100/0 | 100/0 | 20/80 |
| | Reaction temperature (°C) | 280 | 280 | 260 | 250 | 280 | 280 | 260 | 260 | 240 | 240 | 240 |
| | Reaction time (hour) | 10 | 9 | 30 | 31 | 5 | 4 | 1.2 | 1 | 14 | 24 | - |
| Results | Alcohol conversion (%) | 100 | 100 | 99.8 | 99.2 | 100 | 100 | 56 | 62 | 90 | 95 | - |
| | Yield of olefin (%) | 94.4 | 94.7 | 80.5 | 49.1 | 94.6 | 93.9 | 50.3 | 49.5 | 49.7 | 47.9 | - |
| | Yield of dimer (%) | 5.5 | 5.1 | 9.4 | 5.6 | 5.4 | 5.4 | 11.0 | 8.9 | 1.2 | 3.6 | - |

According to the present invention, an olefin may be produced efficiently while suppressing a dimer from being formed.

Example 11

Sulfonation Reaction

Step 1: Sulfonating Step

A 3,000 mL four-neck flask was equipped with a mechanical stirrer and a thermometer, and further equipped with two dropping funnels. After placing the four-neck flask under reduced pressure, the pressure was returned to the atmospheric pressure with nitrogen for nitrogen substitution, and 210 g of 1,4-dioxane (produced by Wako Pure Chemical Industries, Ltd.) and 975 g of chloroform (produced by Wako Pure Chemical Industries, Ltd.) were placed therein and cooled in an ice bath to make the temperature of the solution in the four-neck flask of 5°C or less. After cooling, 56.8 g of sulfuric anhydride (Nisso Sulfan, produced by Nisso Metallochemical Co., Ltd.) was added dropwise thereto from the dropping funnel over 1 hour. After completing the dropwise addition, the mixture was stirred for 0.5 hour. Thereafter, 152 g of the olefin prepared in Example 1 was added dropwise thereto from the dropping funnel over 1 hour. Thereafter, the mixture was stirred for 3 hours while the four-neck flask was cooled in an ice bath.

Step 2: Neutralizing Step

120 g of a 48% by mass sodium hydroxide aqueous solution and 300 g of ion exchanged water were placed in a 3,000 mL SUS beaker and then cooled in an ice bath. After cooling sufficiently, the mixture was stirred with a homomixer while adding the solution obtained in the reaction step gradually thereto under cooling in an ice bath. After completing the addition of the solution obtained in the sulfonating step to the beaker, the mixture was stirred at 5,000 rpm for 3 hours.

Step 3: Hydrolyzing Step

The solution obtained in the neutralizing step was placed in a recovery flask, from which chloroform, 1,4-dioxane and water were distilled off with a rotary evaporator. 670 g of ion exchanged water was added to the resulting concentrated product to prepare an aqueous solution containing a precursor of sodium olefin sulfonate. A 400 g portion of the aqueous solution was placed in a 1 L autoclave and reacted at 160°C for 3 hours, thereby providing a sodium olefin sulfonate aqueous solution.

The resulting sodium olefin sulfonate aqueous solution had an effective ingredient content of 21% by mass. The content of the effective ingredient was measured by a potentiometric titration method using a benzethonium chloride solution (synthetic detergent test method according to JIS K3362).

**Claims**

1. A method for producing an olefin, comprising subjecting a raw material alcohol to dehydration reaction in the presence of an acid catalyst, wherein the acid catalyst contains a metal oxide containing aluminum, the raw material alcohol contains two or more kinds of alcohols having different numbers of carbon atoms each being a primary aliphatic alcohol having 8 or more and 22 or less carbon atoms, and a temperature of the reaction is 200°C or more and 300°C or less, and wherein the dehydration reaction is liquid phase reaction.

2. The method for producing an olefin according to claim 1, wherein the total content of the alcohol that has the first largest content and the alcohol that has the second largest content in the raw material alcohol is 50% by mass or more.

3. The method for producing an olefin according to claim 1 or 2, wherein the alcohol that has the first largest content and the alcohol that has the second largest content in the raw material alcohol are an alcohol (A) and an alcohol (B) having a larger number of carbon atoms than the alcohol (A), and the mass ratio (A/B) of the alcohol (A) and the alcohol (B) is 1/9 or more and 9/1 or less.

4. The method for producing an olefin according to any one of claims 1 to 3, wherein the alcohol that has the first largest content and the alcohol that has the second largest content in the raw material alcohol are an alcohol (A) and an alcohol (B) having a larger number of carbon atoms than the alcohol (A), and the difference in the number of carbon atoms between the alcohol (A) and the alcohol (B) is 1 or more and 4 or less.

5. The method for producing an olefin according to any one of claims 1 to 4, wherein the raw material alcohol is consisted of two kinds of alcohols including an alcohol (A) and an alcohol (B) having a larger number of carbon atoms than the alcohol (A).

6. The method for producing an olefin according to any one of claims 1 to 5, wherein the alcohol that has the first largest content and the alcohol that has the second largest content in the raw material alcohol are an alcohol (A) and an alcohol (B) having a larger number of carbon atoms than the alcohol (A), and the number of carbon atoms of the alcohol (A) is 16 and the number of carbon atoms of the alcohol (B) is 18.

7. The method for producing an olefin according to claim 6, wherein the alcohol (A) is 1-hexadecanol and the alcohol (B) is 1-octadecanol.

8. The method for producing an olefin according to any one of claims 1 to 7, wherein the acid catalyst is a solid acid catalyst.

9. The method for producing an olefin according to any one of claims 1 to 8, wherein the dehydration reaction is performed while removing water produced through the dehydration reaction outside the reaction system.

10. The method for producing an olefin according to any one of claims 1 to 9, wherein the dehydration reaction is performed while introducing an inert gas.

**Patentansprüche**

1. Verfahren zur Erzeugung eines Olefins, enthaltend die Durchführung einer Dehydratisierungsreaktion mit einem Ausgangsmaterialalkohol in der Gegenwart eines sauren Katalysators, worin der saure Katalysator ein Metalloxid enthält, das Aluminium enthält, worin der Ausgangsmaterialalkohol zwei oder mehrere Arten von Alkoholen enthält, die unterschiedliche Zahlen von Kohlenstoffatomen aufweisen, wobei jeder ein primärer aliphatischer Alkohol mit 8 oder mehr und 22 oder weniger Kohlenstoffatomen ist, und wobei eine Temperatur der Reaktion 200°C oder mehr und 300°C oder weniger ist, und wobei es sich bei der Dehydratisierungsreaktion um eine Flüssigphasenreaktion handelt.

2. Verfahren zur Erzeugung eines Olefins gemäß Anspruch 1, worin der Gesamtgehalt des Alkohols, der den größten Gehalt hat, und des Alkohols, der den zweitgrößten Gehalt im Ausgangsmaterialalkohol aufweist, 50 Massen-% oder mehr beträgt.

3. Verfahren zur Erzeugung eines Olefins gemäß Anspruch 1 oder 2, worin der Alkohol, der den größten Gehalt aufweist, und der Alkohol, der den zweitgrößten Gehalt in dem Ausgangsmaterialalkohol aufweist, ein Alkohol (A) und ein Alkohol (B) mit einer größeren Zahl von Kohlenstoffatomen als der Alkohol (A) sind und das Massenverhältnis (A/B) des Alkohols (A) und des Alkohols (B) 1/9 oder mehr und 9/1 oder weniger beträgt.

4. Verfahren zur Erzeugung eines Olefins gemäß mindestens einem der Ansprüche 1 bis 3, worin der Alkohol, der den größten Gehalt aufweist, und der Alkohol, der den zweitgrößten Gehalt in dem Ausgangsmaterialalkohol aufweist, ein Alkohol (A) und ein Alkohol (B) mit einer größeren Zahl von Kohlenstoffatomen als beim Alkohol (A) sind und der Unterschied in der Zahl der Kohlenstoffatome zwischen dem Alkohol (A) und dem Alkohol (B) 1 oder mehr und 4 oder weniger beträgt.

5. Verfahren zur Erzeugung eines Olefins gemäß mindestens einem der Ansprüche 1 bis 4, worin der Ausgangsmaterialalkohol aus zwei Arten von Alkoholen besteht, enthaltend einen Alkohol (A) und einen Alkohol (B) mit einer größeren Zahl von Kohlenstoffatomen als beim Alkohol (A).

6. Verfahren zur Erzeugung eines Olefins gemäß mindestens einem der Ansprüche 1 bis 5, worin der Alkohol, der den größten Gehalt hat, und der Alkohol der den zweitgrößten Gehalt im Ausgangsmaterialalkohol hat, ein Alkohol (A) und ein Alkohol (B) mit einer größeren Zahl von Kohlenstoffatomen als beim Alkohol (A) sind und die Zahl der Kohlenstoffatome des Alkohols (A) 16 und die Zahl der Kohlenstoffatome des Alkohols (B) 18 beträgt.

**7.** Verfahren zur Erzeugung eines Olefins gemäß Anspruch 6, worin der Alkohol (A) 1-Hexadecanol und der Alkohol (B) 1-Octadecanol ist.

**8.** Verfahren zur Erzeugung eines Olefins gemäß mindestens einem der Ansprüche 1 bis 7, worin der saure Katalysator ein fester saurer Katalysator ist.

**9.** Verfahren zur Erzeugung eines Olefins gemäß mindestens einem der Ansprüche 1 bis 8, worin die Dehydratisierungsreaktion durchgeführt wird, während durch die Dehydratisierungsreaktion erzeugtes Wasser aus dem Reaktionssystem entfernt wird.

**10.** Verfahren zur Erzeugung eines Olefins gemäß mindestens einem der Ansprüche 1 bis 9, worin die Dehydratisierungsreaktion durchgeführt wird, während ein Inertgas eingeführt wird.

**Revendications**

**1.** Procédé de production d'une oléfine, comprenant la soumission d'un alcool de matière première à une réaction de déshydratation en présence d'un catalyseur acide, dans lequel le catalyseur acide contient un oxyde métallique contenant de l'aluminium, l'alcool de matière première contient deux types ou plus d'alcools présentant différents nombres d'atomes de carbone, chacun étant un alcool aliphatique primaire présentant 8 atomes de carbone ou plus et 22 atomes de carbone ou moins, et une température de la réaction est de 200°C ou plus et de 300°C ou moins, dans lequel la réaction de déshydratation est une réaction en phase liquide.

**2.** Procédé de production d'une oléfine selon la revendication 1, dans lequel la teneur totale de l'alcool qui présente la première teneur la plus élevée et de l'alcool qui présente la seconde teneur la plus élevée dans l'alcool de matière première est de 50 % en masse ou plus.

**3.** Procédé de production d'une oléfine selon la revendication 1 ou 2, dans lequel l'alcool qui présente la première teneur la plus élevée et l'alcool qui présente la seconde teneur la plus élevée dans l'alcool de matière première sont un alcool (A) et un alcool (B) présentant un plus grand nombre d'atomes de carbone que l'alcool (A), et le rapport massique (A/B) de l'alcool (A) et l'alcool (B) est 1/9 ou plus et 9/1 ou moins.

**4.** Procédé de production d'une oléfine selon l'une quelconque des revendications 1 à 3, dans lequel l'alcool qui présente la première teneur la plus élevée et l'alcool qui présente la seconde teneur la plus élevée dans l'alcool de matière première sont un alcool (A) et un alcool (B) présentant un plus grand nombre d'atomes de carbone que l'alcool (A), et la différence dans le nombre d'atomes de carbone entre l'alcool (A) et l'alcool (B) est 1 ou plus et 4 ou moins.

**5.** Procédé de production d'une oléfine selon l'une quelconque des revendications 1 à 4, dans lequel l'alcool de matière première est constitué de deux types d'alcools incluant un alcool (A) et un alcool (B) présentant un plus grand nombre d'atomes de carbone que l'alcool (A).

**6.** Procédé de production d'une oléfine selon l'une quelconque des revendications 1 à 5, dans lequel l'alcool qui présente la première teneur la plus élevée et l'alcool qui présente la seconde teneur la plus élevée dans l'alcool de matière première sont un alcool (A) et un alcool (B) présentant un plus grand nombre d'atomes de carbone que l'alcool (A), et le nombre d'atomes de carbone de l'alcool (A) est de 16 et le nombre d'atomes de carbone de l'alcool (B) est de 18.

**7.** Procédé de production d'une oléfine selon la revendication 6, dans lequel l'alcool (A) est le 1-hexadécanol et l'alcool (B) est le 1-octadécanol.

**8.** Procédé de production d'une oléfine selon l'une quelconque des revendications 1 à 7, dans lequel le catalyseur acide est un catalyseur acide solide.

**9.** Procédé de production d'une oléfine selon l'une quelconque des revendications 1 à 8, dans lequel la réaction de déshydratation est effectuée en éliminant l'eau produite par la réaction de déshydratation à l'extérieur du système de réaction.

10. Procédé de production d'une oléfine selon l'une quelconque des revendications 1 à 9, dans lequel la réaction de déshydratation est effectuée en introduisant un gaz inerte.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008538206 T
- JP 2008056671 A
- WO 2011052732 A
- JP 2003095994 A
- JP 145452 A

**Non-patent literature cited in the description**

- *Shimadzu Review,* 1991, vol. 48 (1), 35-44